# FASCICULE DE BREVET EUROPEEN

(11) **EP 1 006 153 B1**
(45) Date de publication et mention de la délivrance du brevet: **12.02.2003**
(21) Numéro de dépôt: 99402628.4
(22) Date de dépôt: 22.10.1999
(51) Int. Cl.: C09B 1/20, A61K 7/13

(54) **Amino-di-anthraquinones cationiques, utilisation, compositions de teinture les renfermant et procédés de teinture**
Kationische Aminodianthrachinone, deren Verwendung, diese enthaltende Färbemittel sowie Färbeverfahren
Cationic amino-dianthraquinones, use thereof, dyeing compositions containing them and dyeing processes

(30) Priorité: 30.11.1998 FR 9815045
(43) Date de publication de la demande: 07.06.2000
(73) Titulaire: L'OREAL, 75008 Paris (FR)
(72) Inventeur: Genet, Alain, 93600 Aulnay sous Bois (FR); Lagrange, Alain, 77700 Coupvray (FR)
(74) Mandataire: Dossmann, Gérard

(56) Documents cités:
- DE-A- 2 338 151
- FR-A- 1 379 649
- GB-A- 2 088 417
- US-A- 5 314 505
- US-A- 5 486 629
- PROF. DR. HEYMANN EBERHARD: 'Haut, Haar und Kosmetik', 1994, S.HIRZEL VERLAG, STUTTGART
- VENKATARAMAN K.: 'The Chemistry of Synthetic Dyes Volume V', 1971, ACADEMIC PRESS, NEW YORK
- DR. WILMSMANN H.: 'A Comparison of Hair and Textile Coloring' AMERICAN PARFUMER & AROMATICS pages 41 - 46

## Description

La présente invention concerne des amino-di-anthraquinones cationiques comportant au moins un groupement cationique Z, Z étant choisi parmi des chaînes aliphatiques comportant au moins un cycle insaturé quaternisé, leur utilisation à titre de colorant direct dans les applications de teinture des matières kératiniques humaines, en particulier des fibres kératiniques humaines et notamment les cheveux, les compositions tinctoriales les contenant, ainsi que les procédés de teinture les mettant en oeuvre.

Il est connu de teindre les fibres kératiniques humaines telles que les cheveux, avec des compositions tinctoriales contenant des colorants directs, c'est-à-dire des molécules colorantes ayant une affinité pour lesdites fibres. Le procédé de teinture qui les met en oeuvre est un procédé dit de coloration directe qui consiste à laisser pauser les colorants directs sur les fibres, puis à les rincer.

Les colorations qui en résultent sont des colorations temporaires ou semi-permanentes, car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration.
Parmi les colorants directs connus, on a déjà décrit des aminoanthraquinones cationiques. De telles aminoanthraquinones sont par exemple décrites dans les brevets français N°- 1 422 016 et son addition 87.902, 1 391 675, 1 401 163, 1 379 649, 1 430 089, 1 584 965, 2 050 397, 2 548 895 , les brevets américains N°- 5 169 403, 5 314 505, 5 486 629, 5 520 707, et les brevets européens N°-818 193 et 852 136, et comportent un seul noyau anthraquinonique.

Cependant, en teinture capillaire, on recherche en permanence des colorants directs qui présentent des caractéristiques toujours plus performantes.

C'est donc après d'importantes recherches menées sur la question que la demanderesse vient maintenant de découvrir une nouvelle classe d'aminoanthraquinones cationiques, les amino-di-anthraquinones cationiques comportant au moins un groupement cationique Z, Z étant choisi parmi des chaînes aliphatiques comportant au moins un cycle insaturé quaternisé.
Cette nouvelle famille de colorants présente la particularité très avantageuse d'une plus grande solubilité dans les milieux de teinture et ces nouveaux colorants engendrent des teintures, par coloration directe, dotées d'une puissance et d'une résistance (aux diverses agressions que peuvent subir les cheveux : lumière, intempéries, shampooings, transpiration), notablement améliorée par rapport à celle des teintures réalisées avec des aminoanthraquinones cationiques connues de l'art antérieur.

Cette découverte est à la base de la présente invention.

La présente invention a ainsi pour objet les amino-di-anthraquinones de formule (I) suivante : 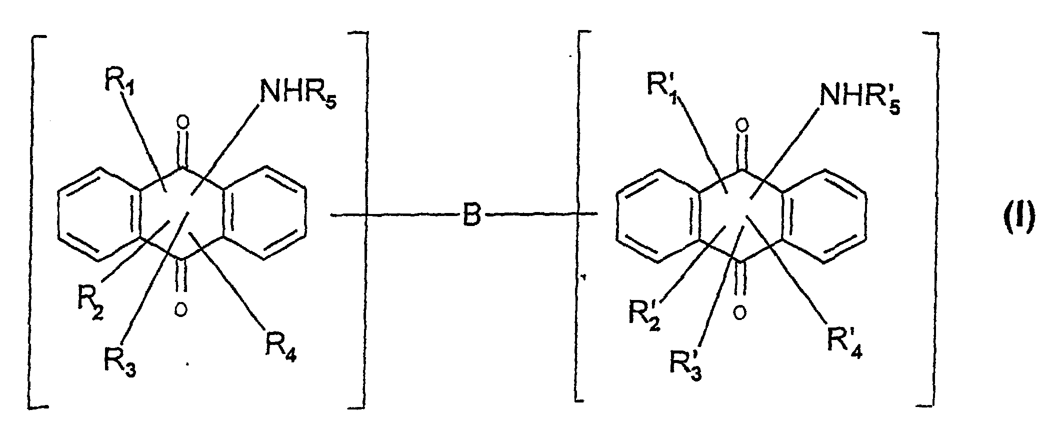 formule dans laquelle,
- **B** est un bras de liaison qui représente une chaîne alkyle comportant de préférence de 1 à 14 atomes de carbone, linéaire ou ramifiée pouvant être interrompue par un ou plusieurs groupements Z (défini(s) ci-après) et/ou par un ou plusieurs hétéroatomes tels que des atomes d'oxygène, de soufre ou d'azote, et éventuellement substituée par un ou plusieurs radicaux hydroxyle ou alcoxy en C₁-C₆, et pouvant porter une ou plusieurs fonctions cétone ;
- **R**_{**1**}**, R**_{**2**}**, R**_{**3**}**, R**_{**4**}**, R'**_{**1**}**, R'**_{**2**}**, R'**_{**3**} **et R'**_{**4**}, qui peuvent être identiques ou différents, représentent l'une des deux valences d'un bras de liaison B, un atome d'hydrogène ; un atome d'halogène ; un groupement Z (défini ci-après) ; un radical alkyl(C₁-C₆) ; un radical monohydroxyalkyle en C₁-C₆ ; un radical polyhydroxyalkyle en C₂-C₆ ; un radical cyano ; un radical nitro ; un radical carboxy ; un radical carbamyle ; un radical sulfo ; un radical amino substitué ou non substitué, ayant les mêmes significations que NHR₅ ou NHR'₅ définis ci-dessous, et pouvant être identique ou différent ; un groupement OR₆ ou SR₆ ou OR'₆ ou SR'₆ défini ci-après.
- **R**_{**5**} **et R'**_{**5**}, identiques ou différents, désignent l'une des deux valences d'un bras de liaison B ; un atome d'hydrogène ; un groupement Z défini ci-après ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alky)(C₁-C₆)carbonylalkyle en C₁-C₆ ; un radical aminoalkyle en C₁-C₆ dont l'alkyle peut être substitué par un ou plusieurs radicaux hydroxy ; un radical aminoalkyle en C₁-C₆ dont l'alkyle peut être substitué par un ou plusieurs radicaux hydroxy et dont l'amine est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₈)carboxy, thiocarbamyle, ou parmi les groupements Z (définis ci-après), ou pouvant former ensemble, avec l'atome d'azote auquel ils sont rattachés, un cycle à 5 ou 6 chaînons, carboné, ou contenant un ou plusieurs hétéroatomes ;
- **R**_{**6**} **et R'**_{**6**}, identiques ou différents, désignent l'une des deux valences d'un bras de liaison B ; un atome d'hydrogène ; un radical alkyle en C₁-C₆; un radical monohydroxyalkyle en C₁-C₆ ; un radical polyhydroxyalkyle en C₂-C₆ ; un groupement Z (défini ci-après) ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-Z-aminosulfonylalkyle(C₁-C₆) ; un radical N-alkyl(C₁-C₆)aminosulfonylalkyle(C₁-C₆) ; un radical N,N-dialkyl(C₁-C₆)aminosulfonylalkyle en C₁-C₆ ; un radical alkyl(C₁-C₆)sulfinyl-alkyle en C₁-C₆ ; un radical alkyl(C₁-C₆)sulfonylalkyle en C₁-C₆ ; un radical alkyl(C₁-C₆)-carbonylalkyle en C₁-C₆ ; un radical aminoalkyle en (C₁-C₆) dont l'alkyle peut être substitué par un ou plusieurs radicaux hydroxy ; un radical aminoalkyle en (C₁-C₆) dont l'alkyle peut être substitué par un ou plusieurs radicaux hydroxy et dont l'amine est substituée par un ou deux radicaux identiques ou différents choisis parmi les radicaux alkyle(C₁-C₆), monohydroxyalkyle(C₁-C₆),polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle, ou parmi les groupements Z (définis ci-après) ou pouvant former ensemble, avec l'atome d'azote auquel ils sont rattachés, un cycle à 5 ou 6 chaînons, carboné, ou contenant un ou plusieurs hétéroatomes ;
- Z est choisi parmi les groupements cationiques insaturés de formules (II) et (III) suivantes: 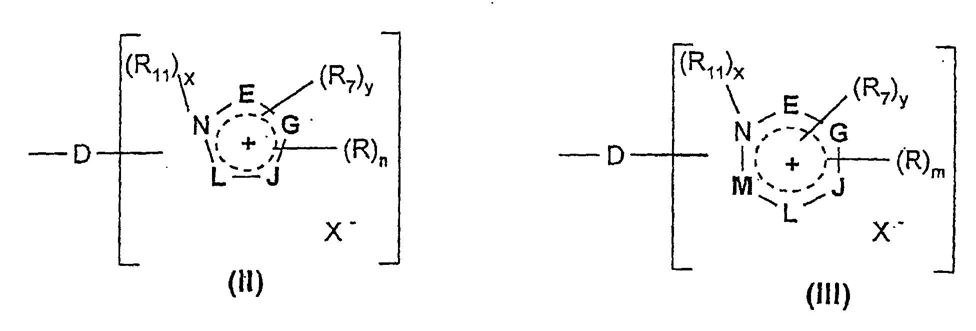 dans lesquelles
   - D est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une ou plusieurs fonctions cétone ;
   - les sommets E, G, J, L et M, identiques ou différents, représentent un atome de carbone, d'oxygène, de soufre ou d'azote ;
   - n est un nombre entier compris entre 0 et 4 inclusivement ;
   - m est un nombre entier compris entre 0 et 5 inclusivement ;
   - les radicaux R, identiques ou différents, représentent l'une des deux valences d'un bras de liaison B, un second groupement Z identique ou différent du premier groupement Z, un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un'radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
- R₇ représente l'une des deux valences d'un bras de liaison B, un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle, un second groupement Z identique ou différent du premier groupement Z ;
- R₁₁ représente l'une des deux valences d'un bras de liaison B, un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
- x et y sont des nombres entiers égaux à 0 ou 1 ; avec les conditions suivantes :
   - dans les groupements cationiques insaturés de formule (II) :
      - lorsque x = 0, le bras de liaison D est rattaché à l'atome d'azote,
      - lorsque x = 1, le bras de liaison D est rattaché à l'un des sommets E, G, J ou L,
      - y ne peut prendre la valeur 1 que :
         1) lorsque les sommets E, G, J et L représentent simultanément un atome de carbone, et que le radical R₇ est porté par l'atome d'azote du cycle insaturé ; ou bien
         2) lorsqu'au moins un des sommets E, G, J et L représente un atome d'azote sur lequel le radical R₇ est fixé ;
   - dans les groupements cationiques insaturés de formule (III) :
      - lorsque x = 0, le bras de liaison D est rattaché à l'atome d'azote,
      - lorsque x = 1, le bras de liaison D est rattaché à l'un des sommets E, G, J, L ou M,
      - y ne peut prendre la valeur 1 que lorsqu'au moins un des sommets E, G, J, L et M représente un atome divalent, et que le radical R₇ est porté par l'atome d'azote du cycle insaturé ;
- X⁻ représente un anion monovalent ou divalent et est de préférence choisi parmi un atome d'halogène tel que le chlore, le brome, le fluor ou l'iode, un hydroxyde, un hydrogènesulfate, ou un alkyl(C₁-C₆)sulfate tel que par exemple un méthylsulfate ou un éthylsulfate ;
**étant entendu que :**
- (i) le nombre de groupement cationique Z est au moins égal à 1,

Dans les formules (I), (II) et (III) ci-dessus les radicaux alkyle et alcoxy peuvent être linéaires ou ramifiés.
Les composés de formule (I) peuvent être éventuellement salifiés par des acides minéraux forts tels que HCI, HBr, H₂SO₄, ou des acides organiques tels qu'acétique, tartrique, lactique, citrique ou succinique.

Parmi les cycles des groupements insaturés Z de formule (II) ci-dessus, on peut notamment citer à titre d'exemple les cycles pyrrolique, imidazolique, pyrazolique, oxazolique, thiazolique et triazolique.

Parmi les cycles des groupements insaturés Z de formule (III) ci-dessus, on peut notamment citer à titre d'exemple les cycles pyridinique, pyrimidinique, pyrazinique, oxazinique et triazinique.

Parmi les amino-di-anthraquinones cationiques de formule (I) on peut notamment citer les composés de formules (I)₂ à (I)₁₂ suivantes :

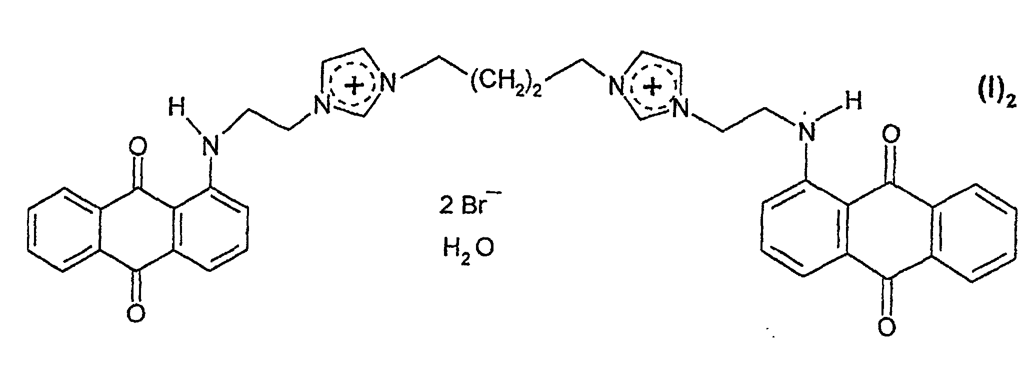

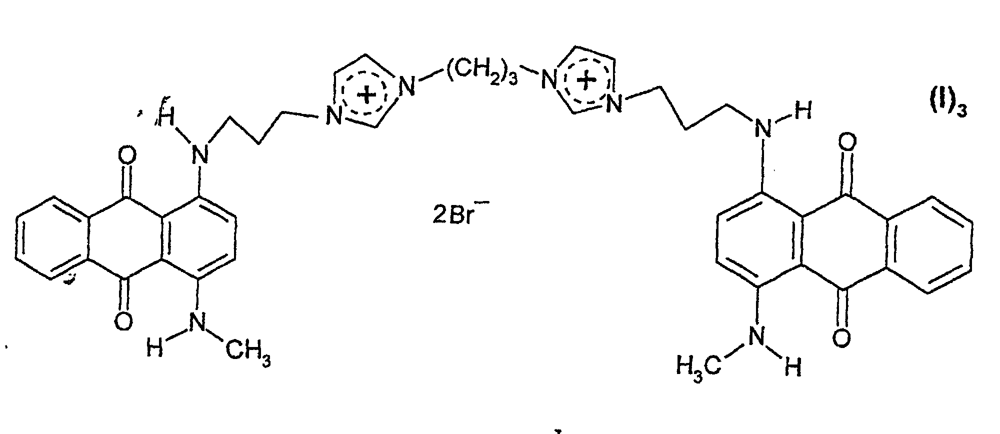

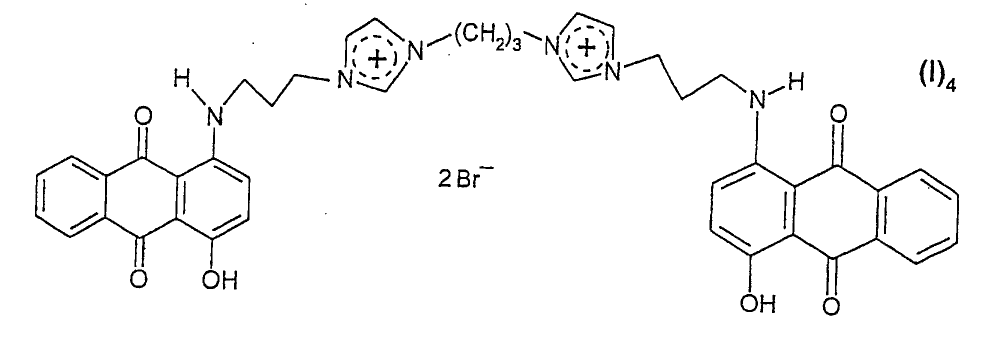

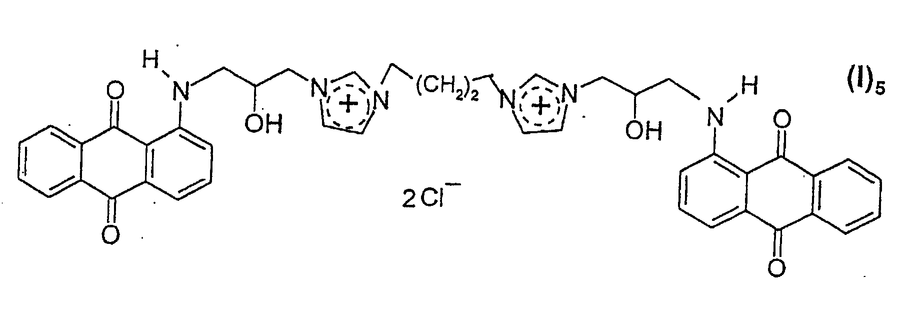

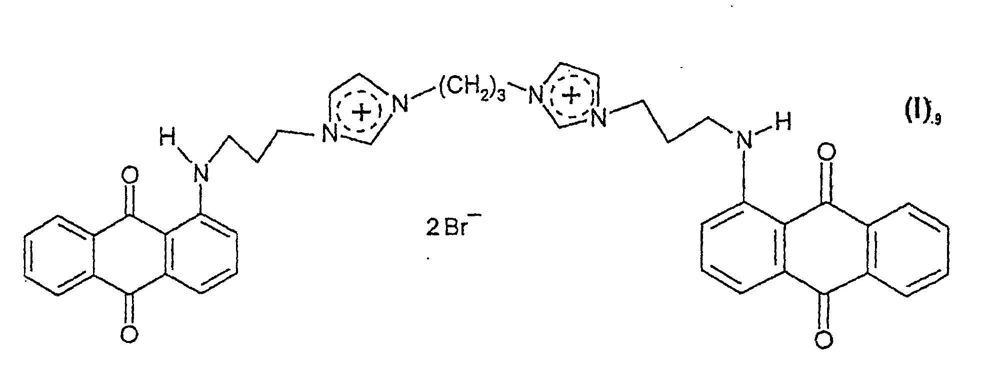

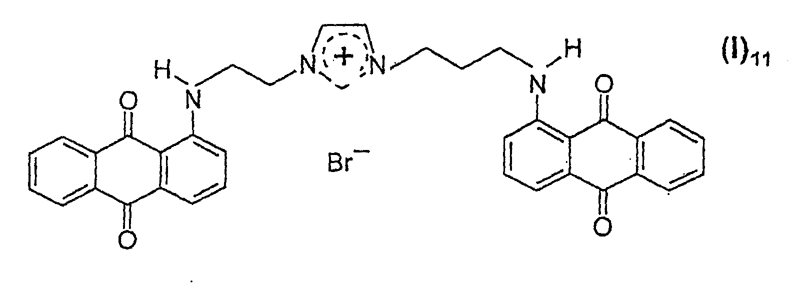

Les amino-di-anthraquinones cationiques de formule (I) conformes à l'invention peuvent être facilement obtenues, selon des procédés généralement bien connus de l'état de la technique et notamment par exemple par :
◆ condensation de deux molécules d'anthraquinone comportant un radical halogénoalkyle sur une molécule d'un composé porteur de deux radicaux amine tertiaire séparés par un bras de liaison B tel que défini dans la formule (I) décrite ci-dessus, ou bien,
◆ condensation de deux molécules d'anthraquinone comportant un radical amine tertiaire sur une molécule d'un composé porteur de deux radicaux halogène séparés par un bras de liaison B tel que défini dans la formule (I) décrite ci-dessus, ou bien,
◆ (a) condensation d'une molécule d'anthraquinone comportant un radical amine tertiaire sur une molécule d'un composé porteur de deux radicaux halogène séparés par un bras de liaison B tel que défini dans la formule (I) décrite ci-dessus, et (b) condensation d'une deuxième molécule d'anthraquinone différente de la première et comportant elle aussi un radical amine tertiaire, ou bien,
◆ (a) condensation d'une molécule d'anthraquinone porteur d'un radical halogénoalkyle sur une molécule d'un composé porteur de deux radicaux amine tertiaire séparés par un bras de liaison B tel que défini dans la formule (I) décrite ci-dessus ,et (b) condensation d'une deuxième molécule d'anthraquinone différente de la première et porteur elle aussi d'un radical halogénoalkyle, ou bien encore,
◆ condensation d'une molécule d'anthraquinone comportant un radical amine tertiaire sur une molécule d'anthraquinone comportant un radical halogénoalkyle.

L'étape de quaternisation est, généralement par commodité, la dernière étape de la synthèse, mais peut intervenir plus tôt dans la suite des réactions conduisant à la préparation des composés de formule (I).

L'invention a aussi pour objet une composition de teinture pour matières kératiniques humaines, en particulier pour fibres kératiniques humaines, et plus particulièrement les cheveux comprenant, dans un milieu approprié pour la teinture, une quantité efficace d'au moins une amino-di-anthraquinone cationique de formule (I) ci-avant définie.

L'invention a pour autre objet l'utilisation d'amino-di-anthraquinones cationiques de formule (I), 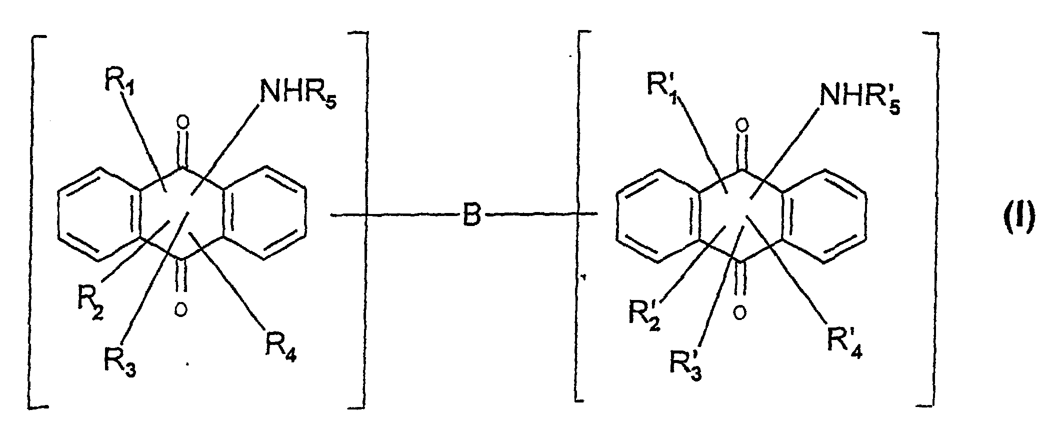 pour laquelle B, R₁, R₂, R₃, R₄, R₅, R'₁, R'₂, R'₃, R'₄, et R'₅ ont les mêmes significations que précédemment décrit, étant entendu que le nombre de groupement cationique Z est au moins égal à 1,
à titre de colorants directs, dans des, ou pour la préparation de, compositions tinctoriales pour matières kératiniques humaines et en particulier pour fibres kératiniques humaines telles que les cheveux.

Mais d'autres caractéristiques, aspects et avantages de l'invention apparaîtront encore plus clairement à la lecture de la description qui va suivre, ainsi que des divers exemples concrets, mais nullement limitatifs, destinés à l'illustrer. La ou les amino-di-anthraquinone(s) cationique(s) de formule (I) conformes à l'invention et/ou le ou leurs sels d'addition avec un acide représentent de préférence de 0,005 à 12 % en poids environ du poids total de la composition tinctoriale, et encore plus préférentiellement de 0,05 à 6 % en poids environ de ce poids.

Les amino-di-anthraquinones cationiques de formule (I) conformes à l'invention peuvent également servir, dans les procédés bien connus de teinture d'oxydation, utilisant des colorants d'oxydation (précurseurs de colorants d'oxydation et éventuellement des coupleurs), à nuancer ou enrichir de reflets les teintures obtenues avec les colorants d'oxydation.

La composition tinctoriale selon l'invention peut encore contenir, pour élargir la palette de nuances et obtenir des teintes variées, outre les amino-di-anthraquinones cationiques de formule (I) selon l'invention, d'autres colorants directs classiquement utilisés, et notamment, des colorants nitrés benzéniques, comme les nitrophénylènediamines, les nitrodiphénylamines, les nitroanilines, les éthers de phénol nitrés ou les nitrophénols, des nitropyridines, d'autres colorants anthraquinoniques différant de ceux conformes à la présente invention, des colorants mono- ou di-azoïques, triarylméthaniques, aziniques, acridiniques et xanthéniques, ou encore des colorants métallifères.

La proportion de tous ces autres colorants directs d'addition peut varier entre 0,5 et 10% en poids environ par rapport au poids total de la composition tinctoriale.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, ainsi que les alcools aromatiques comme l'alcool benzylique ou le phénoxyéthanol, les produits analogues et leurs mélanges.
Les solvants peuvent être présents dans des proportions de préférence comprises entre 1 et 40% en poids par rapport au poids total de la composition tinctoriale, et plus préférentiellement encore entre 5 et 30% en poids environ.
On peut également ajouter à la composition selon l'invention des amides gras tels que les mono- et di-éthanolamides des acides dérivés du coprah, de l'acide laurique ou de l'acide oléïque, à des concentrations comprises entre environ 0,05 et 10% en poids.
On peut encore ajouter à la composition selon l'invention des agents tensio-actifs bien connus de l'état de la technique et de type anionique, cationique, non-ionique, amphotère, zwittérionique ou leurs mélanges, de préférence en une proportion comprise entre environ 0,1 et 50% en poids et avantageusement entre environ 1 et 20% en poids par rapport au poids total de la composition.
On peut également utiliser des agents épaississants dans une proportion allant d'environ 0,2 à 5%.
Ladite composition tinctoriale peut contenir en outre divers adjuvants usuels tels que des agents anti-oxydants, des parfums, des agents séquestrants, des agents dispersants, des agents de conditionnement du cheveu, des agents conservateurs, des agents opacifiants, ainsi que tout autre adjuvant utilisé habituellement en teinture des matières kératiniques humaines.
Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des matières kératiniques humaines.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium, et les composés de formule (V) suivante : 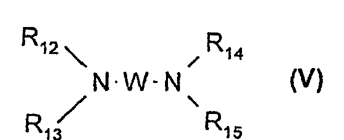 dans laquelle W est un reste propylène éventuellement substitué par un groupement hydroxyle ou un radical alkyle en C₁-C₆ ; R₁₂, R₁₃, R₁₄ et R₁₅, identiques ou différents, représentent un atome d'hydrogène, un radical alkyle en C₁-C₆ ou hydroxyalkyle en C₁-C₆.

La composition tinctoriale selon l'invention peut se présenter sous des formes diverses, telles que sous forme de liquides, de crèmes, de gels, ou sous toute autre forme appropriée pour réaliser une teinture des matières kératiniques humaines et plus particulièrement des fibres kératiniques humaines et notamment des cheveux. En particulier, elle peut être conditionnée sous pression en flacon aérosol en présence d'un agent propulseur et former une mousse.

Un autre objet de la présente invention porte sur un procédé de teinture des fibres kératiniques humaines et en particulier des cheveux, par coloration directe, consistant à laisser agir une composition tinctoriale renfermant au moins une amino-di-anthraquinone cationique de formule (I) sur les fibres sèches ou humides.
On peut utiliser la composition selon l'invention en tant que composition non rincée, c'est-à-dire qu'après application de la composition sur les fibres, on sèche sans rinçage intermédiaire.
Dans les autres modes d'application, on laisse agir la composition sur les fibres pendant un temps de pose variant entre 3 et 60 minutes environ, de préférence entre 5 et 45 minutes environ, on rince, éventuellement on lave, puis on rince à nouveau, et on sèche.

Des exemples concrets et non limitatifs illustrant l'invention vont maintenant être donnés.

### EXEMPLE DE PREPARATION

### EXEMPLE 1

### Synthèse du composé de formule (I)₂

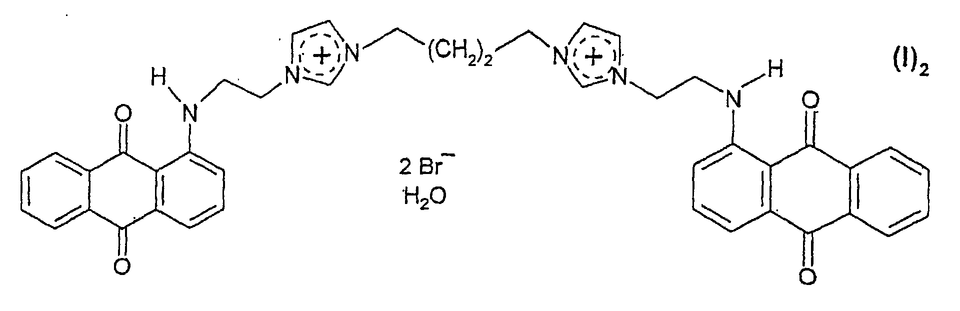

On a chauffé au reflux pendant 7 heures 33,0g (0,1 mole) de 1-(2-bromoéthylamino)-anthraquinone (RN 3591-05-7) et 10,2g (0,05mole) de 1,4-diimidazol-1-yl-butane (RN 69506-86-1) dans 180ml d'isobutanol (disparition de l'anthraquinone de départ en chromatographie sur couche mince).

La suspension obtenue a été refroidie à température ambiante, diluée avec 180ml d'éthanol absolu, et essorée.

Après purification par recristallisation de l'éthanol à 90 au reflux séchage à 50°C sous vide en présence d'anhydride phosphorique on a obtenu 32,8g de cristaux rouges fondant à 224-225°C (Kofler) et dont l'analyse élémentaire calculée pour C₄₂H₃₈N₆O₄Br₂ + H₂O était :

| **%** | **C** | **H** | **N** | **O** | **Br** |
|---|---|---|---|---|---|
| théorie | 58,08 | 4,64 | 9,68 | 9,21 | 18,40 |
| trouvé | 58,28 | 4,68 | 9,45 | 9,10 | 18,46 |

### EXEMPLE DE COMPOSITIONS TINCTORIALES

### EXEMPLE 2

On a préparé la composition de teinture réunie dans le tableau suivant : *(toutes teneurs exprimées en grammes - M.A. désigne Matière Active)*

| | |
|---|---|
| | Exemple 2 |
| colorant de formule (I)₂ | 0,434 |
| Hydroxyéthylcellulose vendue sous la dénomination NATROSOL 250 MR par la société Aqualon | 0,72 |
| Alcool benzylique | 4 |
| Polyéthylèneglycol à 6 oxyde d'éthylène | 6 |
| Alkyl(C8-C10) polyglucoside en solution aqueuse à 60% M.A. vendu sous la dénomination ORAMIX CG 110 par la société Seppic | 3 M.A. |
| Tampon phosphate pH 7 q.s.p. | 100 |
| Tampon phosphate pH 9 (acide borique/chlorure de potassium/hydroxyde de sodium q.s.p. | |

On a appliqué la composition ci-dessus sur des mèches de cheveux gris naturels ou permanentés à 90% de blancs et on a laissé poser pendant 20 minutes. Après rinçage à l'eau courante et séchage, les cheveux ont été teints dans une nuance qui est exprimée dans le tableau ci-dessous.

| | |
|---|---|
| Composition de l'exemple 2 | Cuivré irisé |

## Revendications

1. Amino-di-anthraquinones cationiques de formule (I) et leurs sels d'addition avec un acide 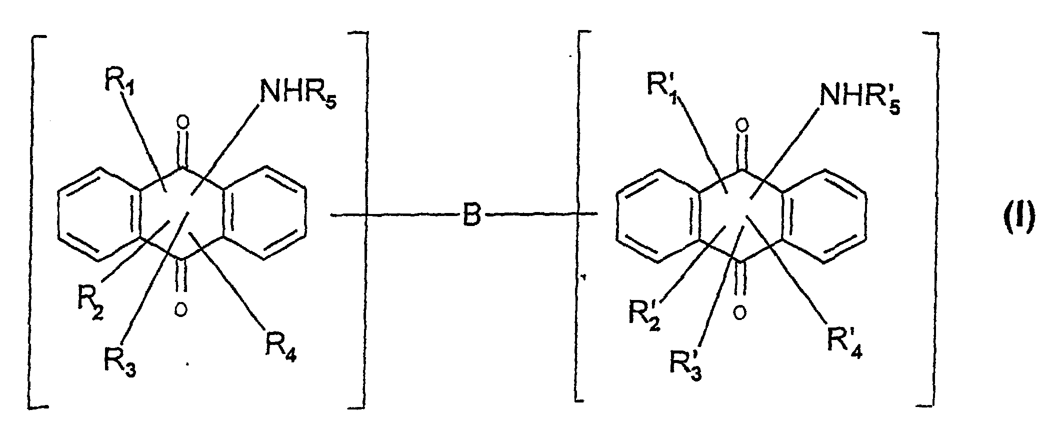 formule dans laquelle,
• **B** est un bras de liaison qui représente une chaîne alkyle comportant de préférence de 1 à 14 atomes de carbone, linéaire ou ramifiée pouvant être interrompue par un ou plusieurs groupements Z (défini(s) ci-après) et/ou par un ou plusieurs hétéroatomes tels que des atomes d'oxygène, de soufre ou d'azote, et éventuellement substituée par un ou plusieurs radicaux hydroxyle ou alcoxy en C₁-C₆, et pouvant porter une ou plusieurs fonctions cétone ;
• **R**_{**1**}**, R**_{**2**}**, R**_{**3**}**, R**_{**4**}**, R'**_{**1**}**, R'**_{**2**}**, R'**_{**3**} **et R'**_{**4**}, qui peuvent être identiques ou différents, représentent l'une des deux valences d'un bras de liaison B, un atome d'hydrogène ; un atome d'halogène ; un groupement Z (défini ci-après) ; un radical alkyl(C₁-C₆) ; un radical monohydroxyalkyle en C₁-C₆ ; un radical polyhydroxyalkyle en C₂-C₆ ; un radical cyano ; un radical nitro ; un radical carboxy ; un radical carbamyle ; un radical sulfo ; un radical amino substitué ou non substitué, ayant les mêmes significations que NHR₅ ou NHR'₅ définis ci-dessous, et pouvant être identique ou différent ; un groupement OR₆ ou SR₆ ou OR'₆ ou SR'₆ défini ci-après.
• **R**_{**5**} et **R'**_{**5**}, identiques ou différents, désignent l'une des deux valences d'un bras de liaison B ; un atome d'hydrogène ; un groupement Z défini ci-après ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkylC₁-C₆)carbonylalkyle en C₁-C₆ ; un radical aminoalkyle en C₁-C₆ dont l'alkyle peut être substitué par un ou plusieurs radicaux hydroxy ; un radical aminoalkyle en C₁-C₆ dont l'alkyle peut être substitué par un ou plusieurs radicaux hydroxy et dont l'amine est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle, ou parmi les groupements Z (définis ci-après), ou pouvant former ensemble, avec l'atome d'azote auquel ils sont rattachés, un cycle à 5 ou 6 chaînons, carboné, ou contenant un ou plusieurs hétéroatomes ;
• **R**_{**6**} **et R'**_{**6**}, identiques ou différents, désignent l'une des deux valences d'un bras de liaison B ; un atome d'hydrogène ; un radical alkyle en C₁-C₆; un radical monohydroxyalkyle en C₁-C₆ ; un radical polyhydroxyalkyle en C₂-C₆ ; un groupement Z (défini ci-après) ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-Z-aminosulfonylalkyle(C₁-C₆) ; un radical N-alkyl(C₁-C₆)aminosulfonylalkyle(C₁-C₆) ; un radical N,N-dialkyl(C₁-C₆)aminosulfonylalkyle en C₁-C₆ ; un radical alkyl(C₁-C₆)sulfinyl-alkyle en C₁-C₆ ; un radical alkyl(C₁-C₆)sulfonylalkyle en C₁-C₆ ; un radical alkyl(C₁-C₆)-carbonylalkyle en C₁-C₆ ; un radical aminoalkyle en (C₁-C₆) dont l'alkyle peut être substitué par un ou plusieurs radicaux hydroxy ; un radical aminoalkyle en (C₁-C₆) dont l'alkyle peut être substitué par un ou plusieurs radicaux hydroxy et dont l'amine est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle, ou parmi les groupements Z (définis ci-après) ou pouvant former ensemble, avec l'atome d'azote auquel ils sont rattachés, un cycle à 5 ou 6 chaînons, carboné, ou contenant un ou plusieurs hétéroatomes ;
• Z est choisi parmi les groupements cationiques insaturés de formules (II) et (III) suivantes, et les groupements cationiques saturés de formule (IV) suivante : 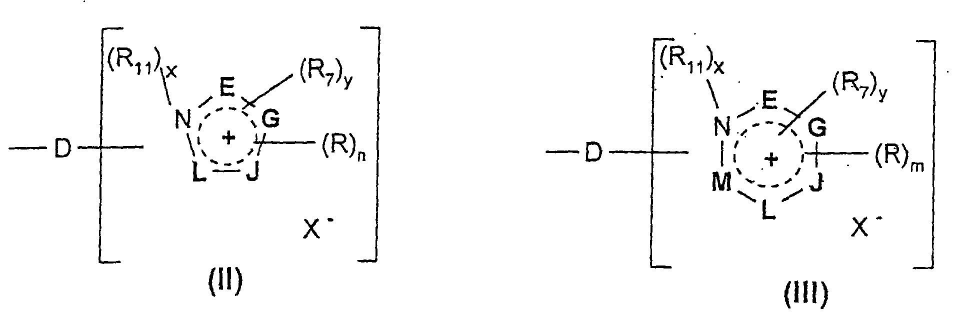 dans lesquelles :
• D est un bras de liaison qui représente une chaîne alkyle comportant de préférence de 1 à 14 atomes de carbone, linéaire ou ramifiée, pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une ou plusieurs fonctions cétone ;
• les sommets E, G, J, L et M, identiques ou différents, représentent un atome de carbone, d'oxygène, de soufre ou d'azote ;
• n est un nombre entier compris entre 0 et 4 inclusivement ;
• m est un nombre entier compris entre 0 et 5 inclusivement ;
• les radicaux R, identiques ou différents, représentent l'une des deux valences d'un bras de liaison B, un second groupement Z identique ou différent du premier groupement Z, un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
• R₇ représente l'une des deux valences d'un bras de liaison B, un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle, un second groupement Z identique ou différent du premier groupement Z ;
• R₁₁ représente l'une des deux valences d'un bras de liaison B, un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
• x et y sont des nombres entiers égaux à 0 ou 1 ; avec les conditions suivantes:
- dans les groupements cationiques insaturés de formule (II) :
- lorsque x = 0, le bras de liaison D est rattaché à l'atome d'azote,
- lorsque x = 1, le bras de liaison D est rattaché à l'un des sommets E, G, J ou L,
- y ne peut prendre la valeur 1 que :
1) lorsque les sommets E, G, J et L représentent simultanément un atome de carbone, et que le radical R₇ est porté par l'atome d'azote du cycle insaturé ; ou bien
2) lorsqu'au moins un des sommets E, G, J et L représente un atome d'azote sur lequel le radical R₇ est fixé ;
- dans les groupements cationiques insaturés de formule (III) :
- lorsque x = 0, le bras de liaison D est rattaché à l'atome d'azote,
- lorsque x = 1, le bras de liaison D est rattaché à l'un des sommets E, G, J, L ou M,
- y ne peut prendre la valeur 1 que lorsqu'au moins un des sommets E, G, J, L et M représente un atome divalent, et que le radical R₇ est porté par l'atome d'azote du cycle insaturé ;
• X⁻ représente un anion monovalent ou divalent,
**étant entendu que :**
- (i) le nombre de groupement cationique Z est au moins égal à 1.

2. Amino-di-anthraquinones cationiques selon la revendication 1, **caractérisée par le fait que** les cycles des groupements insaturés Z de formule (II) sont choisis parmi les cycles pyrrolique, imidazolique, pyrazolique, oxazolique, thiazolique et triazolique.

3. Amino-di-anthraquinones cationiques selon la revendication 1, **caractérisée par le fait que** les cycles des groupements insaturés Z de formule (III) sont choisis parmi les cycles pyridinique, pyrimidinique, pyrazinique, oxazinique et triazinique.

4. Amino-di-anthraquinones cationiques selon l'une quelconque des revendications précédentes, **caractérisées par le fait que** X⁻ est choisi parmi un atome d'halogène, un hydroxyde, un hydrogènesulfate, ou un alkyl(C₁-C₆)sulfate.

5. Amino-di-anthraquinones cationiques selon l'une quelconque des revendications précédentes, **caractérisées par le fait qu'**elles sont choisies parmi celles de formule (I)₂ à (I)₁₂ suivantes :

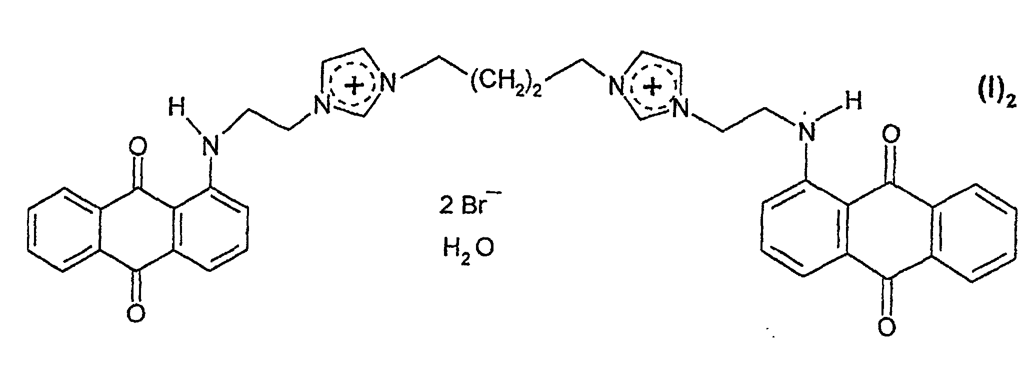

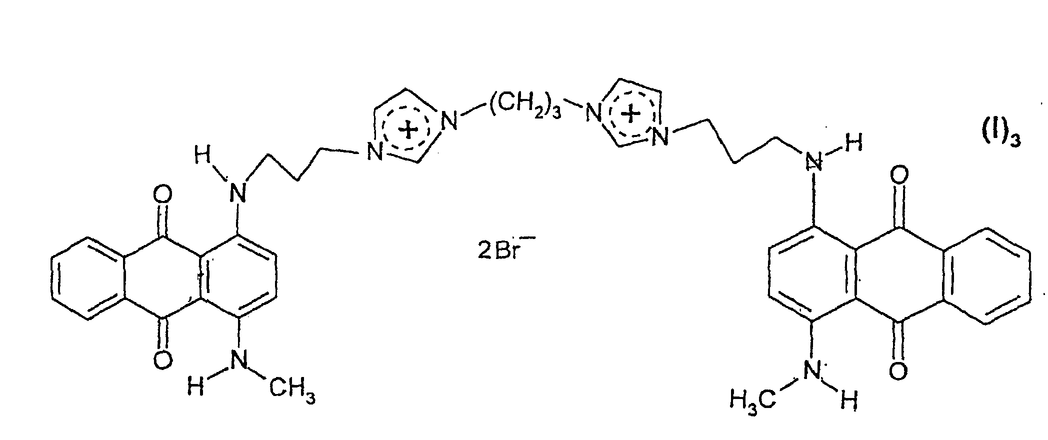

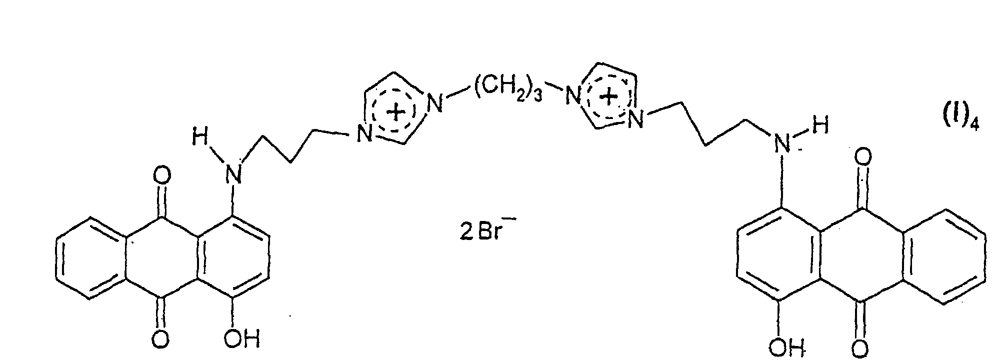

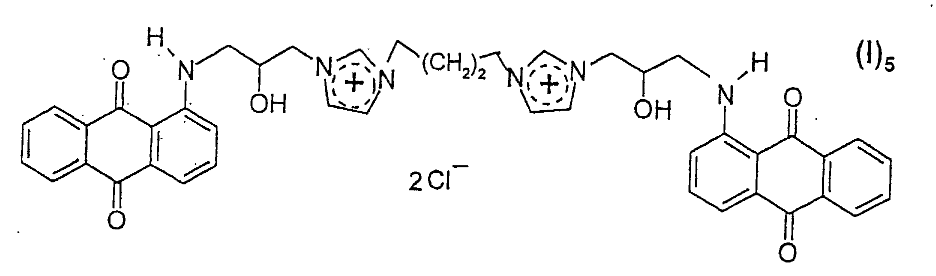

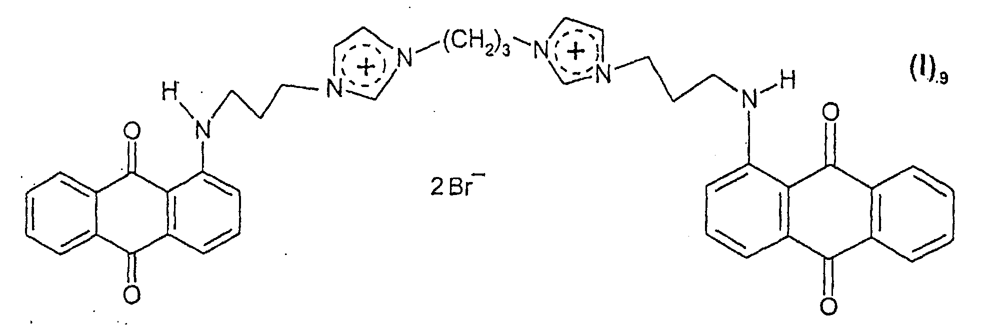

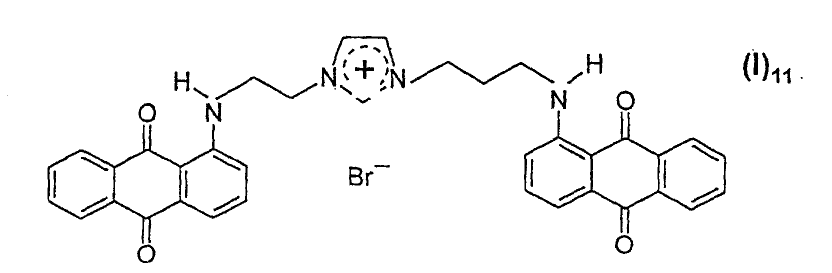

6. Amino-di-anthraquinones cationiques selon l'une quelconque des revendications précédentes, **caractérisées par le fait que** les sels d'addition avec un acide sont choisis parmi les chlorhydrates, les bromhydrates, les sulfates, les citrates, les succinates, les tartrates, les lactates et les acétates.

7. Utilisation d'amino-di-anthraquinones cationiques de formule (I) suivante : 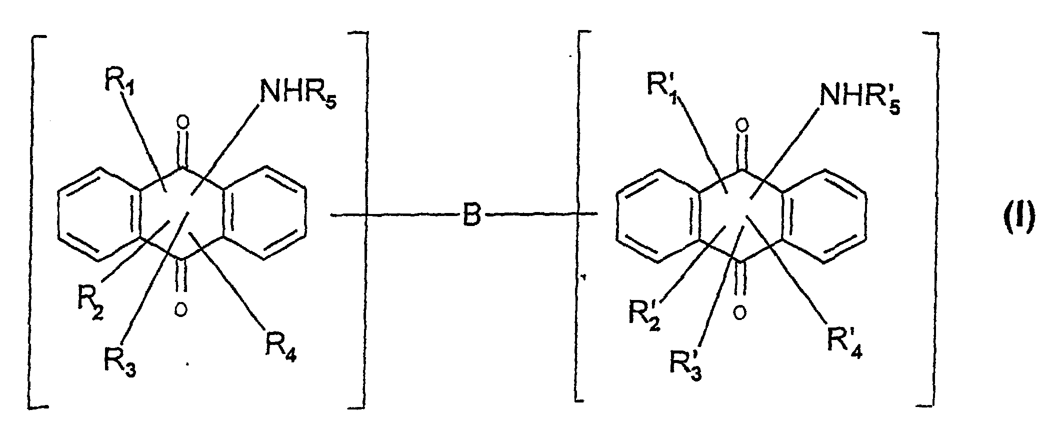 pour laquelle B, R₁, R₂, R₃, R₄, R₅, R'₁, R'₂, R'₃, R'₄, et R'₅ ont les mêmes significations que dans la revendication 1,
à titre de colorants directs dans des, ou pour la fabrication de, compositions tinctoriales pour matières kératiniques humaines, en particulier pour fibres kératiniques humaines et plus particulièrement les cheveux.

8. Composition de teinture pour matières kératiniques humaines, en particulier pour fibres kératiniques humaines, et plus particulièrement les cheveux, **caractérisée par le fait qu'**elle contient, dans un milieu approprié pour la teinture, une quantité efficace d'au moins une amino-di-anthraquinone cationique de formule (I) définie à l'une quelconque des revendications 1 à 7.

9. Composition selon la revendication 8, **caractérisée par le fait qu'**elle a un pH compris entre 3 et 12.

10. Composition selon l'une quelconque des revendications 8 à 9, **caractérisée par le fait que** les amino-di-anthraquinones cationiques sont présentes dans une concentration allant de 0,005 à 12% en poids par rapport au poids total de la composition.

11. Composition selon la revendication 10, **caractérisée par le fait que** les amino-di-anthraquinones cationiques sont présentes dans une concentration allant de 0,05 à 6% en poids par rapport au poids total de la composition.

12. Composition selon l'une quelconque des revendications 8 à 11, **caractérisée par le fait qu'**elle contient d'autres colorants directs, choisis parmi des colorants nitrés benzéniques comme les nitrophénylènediamines, les nitrodiphénylamines, les nitroanilines, les éthers de phénols nitrés ou les nitrophénols, des nitropyridines, des colorants anthraquinoniques autres que ceux de formule (I), des colorants mono- ou di-azoïques, triarylméthaniques, aziniques, acridiniques, xanthéniques, ou des colorants métallifères.

13. Composition selon l'une quelconque des revendications 8 à 12, **caractérisée par le fait que** le milieu approprié pour la teinture est un milieu aqueux constitué par de l'eau et/ou des solvants organiques choisis parmi les alcools, les glycols et les éthers de glycol, dans des proportions comprises entre 1 et 40% en poids par rapport au poids total de la composition.

14. Procédé de teinture des fibres kératiniques humaines et en particulier les cheveux, par coloration directe, **caractérisé par le fait qu'**on applique la composition tinctoriale définie à l'une quelconque des revendications 8 à 13, sur les fibres sèches ou humides, et qu'on sèche ces fibres sans rinçage intermédiaire.

15. Procédé de teinture des fibres kératiniques humaines et en particulier les cheveux, par coloration directe, **caractérisé par le fait qu'**on applique la composition tinctoriale définie à l'une quelconque des revendications 8 à 13, sur les fibres sèches ou humides, et qu'après avoir laissé agir la composition pendant 3 à 60 minutes environ, on rince les fibres, on les lave éventuellement, on les rince à nouveau puis on les sèche.

## Claims

1. Cationic aminodianthraquinones of formula (I) and their addition salts with an acid: 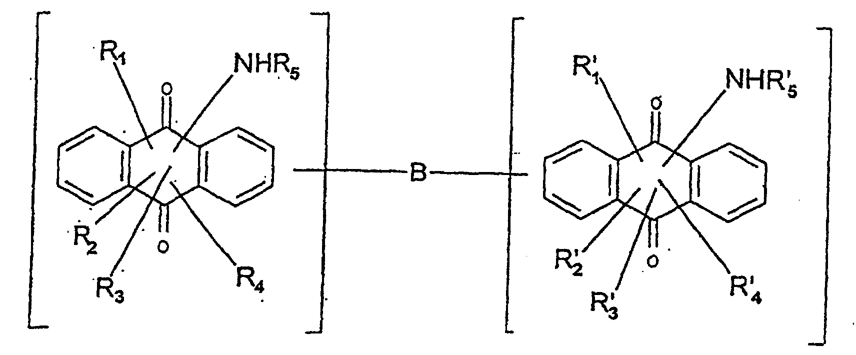 in which formula,
• **B** is a connecting arm which represents a linear or branched alkylene chain preferably comprising from 1 to 14 carbon atoms which can be interrupted by one or more Z groups (defined below) and/or by one or more heteroatoms, such as oxygen, sulphur or nitrogen atoms, which chain is optionally substituted by one or more hydroxyl or C₁-C₆ alkoxy radicals and can carry one or more ketone functional groups;
• **R**_{**1**}**, R**_{**2**}**, R**_{**3**}**, R**_{**4**}**, R'**_{**1**}**, R'**_{**2**}**, R'**_{**3**} and **R'**_{**4**}**,** which can be identical or different, represent one of the two valences of a connecting arm B; a hydrogen atom; a halogen atom; a Z group (defined below); a (C₁-C₆)alkyl radical; a monohydroxy (C₁-C₆)alkyl radical; a polyhydroxy(C₂-C₆)alkyl radical; a cyano radical; a nitro radical; a carboxyl radical; a carbamoyl radical; a sulpho radical; a substituted or unsubstituted amino radical having the same meanings as NHR₅ or NHR'₅ defined below and which may be identical or different; or an OR₆ or SR₆ or OR'₆ or SR'₆ group defined below;
• **R**_{**5**} and **R'**_{**5**}, which are identical or different, denote one of the two valences of a connecting arm B; a hydrogen atom; a Z group defined below; a C₁-C₆ alkyl radical; a monohydroxy(C₁-C₆)alkyl radical; a polyhydroxy(C₂-C₆)alkyl radical; a (C₁-C₆)alkoxy(C₁-C₆)alkyl radical; a benzyl radical; a cyano(C₁-C₆)alkyl radical; a carbamoyl (C₁-C₆)alkyl radical; an N-[(C₁-C₆)alkyl]carbamoyl(C₁-C₆)alkyl radical; an N,N-di[(C₁-C₆)alkyl] carbamoyl(C₁-C₆)alkyl radical; a thiocarbamoyl(C₁-C₆)alkyl radical; a trifluoro (C₁-C₆)alkyl radical; a sulpho(C₁-C₆)alkyl radical; a (C₁-C₆)alkylcarboxy(C₁-C₆)alkyl radical; a (C₁-C₆)alkylsulphinyl(C₁-C₆)alkyl radical; an aminosulphonyl(C₁-C₆)alkyl radical; an N-Z-aminosulphonyl (C₁-C₆)alkyl radical; an N-[(C₁-C₆)alkyl]-aminosulphonyl (C₁-C₆)alkyl radical; an N,N-di[(C₁-C₆)alkyl]aminosulphonyl(C₁-C₆)alkyl radical; a (C₁-C₆)alkylcarbonyl(C₁-C₆)alkyl radical; an amino(C₁-C₆)alkyl radical, the alkyl of which can be substituted by one or more hydroxyl radicals; an amino(C₁-C₆)alkyl radical, the alkyl of which can be substituted by one or more hydroxyl radicals and the amine of which is substituted by one or two identical or different radicals chosen from C₁-C₆ alkyl, monohydroxy(C₁-C₆)alkyl, polyhydroxy(C₂-C₆)alkyl, (C₁-C₆)alkylcarbonyl, carbamoyl, N-[(C₁-C₆)alkyl]-carbamoyl or N,N-di[(C₁-C₆)alkyl]carbamoyl, (C₁-C₆) alkylsulphonyl, formyl, trifluoro (C₁-C₆) alkylcarbonyl, (C₁-C₆)alkylcarboxyl or thiocarbamoyl radicals or from Z groups (defined below) or which can together form, with the nitrogen atom to which they are attached, a 5- or 6-membered carbonaceous ring or one comprising one or more heteroatoms;
• **R**_{**6**} and **R'**_{**6**}**,** which are identical or different, denote one of the two valences of a connecting arm B; a hydrogen atom; a C₁-C₆ alkyl radical; a monohydroxy(C₁-C₆)alkyl radical; a polyhydroxy(C₂-C₆)alkyl radical; a Z group (defined below); a (C₁-C₆)alkoxy(C₁-C₆)alkyl radical; an aryl radical; a benzyl radical; a carboxy(C₁-C₆)alkyl radical; a (C₁-C₆)alkylcarboxy(C₁-C₆)alkyl radical; a cyano(C₁-C₆)alkyl radical; a carbamoyl(C₁-C₆)alkyl radical; an N- [(C₁-C₆)alkyl]carbamoyl(C₁-C₆)alkyl radical; an N,N-di[(C₁-C₆)alkyl]carbamoyl(C₁-C₆)alkyl radical; a trifluoro(C₁-C₆)alkyl radical; an aminosulphonyl(C₁-C₆)alkyl radical; an N-Z-aminosulphonyl(C₁-C₆)alkyl radical; an N-[(C₁-C₆)alkylaminosulphonyl(C₁-C₆)alkyl radical; an N,N-di[(C₁-C₆)alkyl]aminosulphonyl(C₁-C₆)alkyl radical; a (C₁-C₆)alkylsulphinyl(C₁-C₆)alkyl radical; a (C₁-C₆)alkylsulphonyl(C₁-C₆)alkyl radical; a (C₁-C₆)alkylcarbonyl(C₁-C₆)alkyl radical; an amino(C₁-C₆)alkyl radical, the alkyl of which can be substituted by one or more hydroxyl radicals; an amino(C₁-C₆)alkyl radical, the alkyl of which can be substituted by one or more hydroxyl radicals and the amine of which is substituted by one or two identical or different radicals chosen from (C₁-C₆)alkyl, monohydroxy(C₁-C₆)alkyl, polyhydroxy(C₂-C₆)alkyl, (C₁-C₆)alkylcarbonyl, formyl, trifluoro(C₁-C₆)alkylcarbonyl, (C₁-C₆)alkylcarboxyl, carbamoyl, N-[(C₁-C₆)alkyl]carbamoyl, N,N-di[(C₁-C₆)alkyl]-carbamoyl, thiocarbamoyl or (C₁-C₆)alkylsulphonyl radicals or from Z groups (defined below) or which can together form, with the nitrogen atom to which they are attached, a 5- or 6-membered carbonaceous ring or one comprising one or more heteroatoms;
• **Z** is chosen from the unsaturated cationic groups of following formulae (II) and (III): 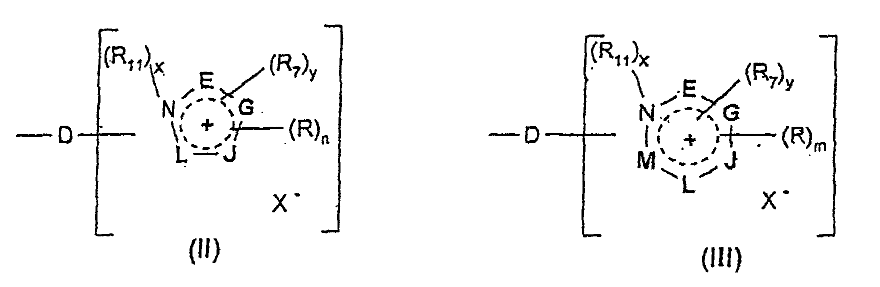 in which:
• D is a connecting arm which represents a linear or branched alkylene chain preferably comprising from 1 to 14 carbon atoms, which chain can be interrupted by one or more heteroatoms, such as oxygen, sulphur or nitrogen atoms, can be substituted by one or more hydroxyl or C₁-C₆ alkoxy radicals and can carry one or more ketone functional groups;
• the ring members E, G, J, L and M, which are identical or different, represent a carbon, oxygen, sulphur or nitrogen atom;
• n is an integer between 0 and 4 inclusive;
• m is an integer between 0 and 5 inclusive;
• the R radicals, which are identical or different, represent one of the two valences of a connecting arm B, a second Z group identical to or different from the first Z group, a halogen atom, a hydroxyl radical, a C₁-C₆ alkyl radical, a monohydroxy (C₁-C₆)alkyl radical, a polyhydroxy(C₂-C₆)alkyl radical, a nitro radical, a cyano radical, a cyano(C₁-C₆)alkyl radical, a (C₁-C₆)alkoxy radical, a tri(C₁-C₆)alkylsilyl(C₁-C₆)alkyl radical, an amido radical, an aldehydo radical, a carboxyl radical, a (C₁-C₆)alkylcarbonyl radical, a thiol radical, a thiol(C₁-C₆)alkyl radical, a (C₁-C₆)alkylthio radical, an amino radical, an amino radical protected by a (C₁-C₆)alkylcarbonyl, carbamoyl or (C₁-C₆)alkylsulphonyl radical; an NHR'' or NR''R''' group in which R'' and R''', which are identical or different, represent a C₁-C₆ alkyl radical, a monohydroxy(C₁-C₆)alkyl radical or a polyhydroxy(C₂-C₆)alkyl radical;
• **R**_{**7**} represents one of the two valences of a connecting arm B, a C₁-C₆ alkyl radical, a monohydroxy(C₁-C₆)alkyl radical, a polyhydroxy(C₂-C₆)alkyl radical, a cyano(C₁-C₆)alkyl radical, a tri(C₁-C₆)alkylsilyl(C₁-C₆)alkyl radical, a (C₁-C₆)alkoxy(C₁-C₆)alkyl radical, a carbamoyl(C₁-C₆)alkyl radical, a (C₁-C₆)alkylcarboxy (C₁-C₆)alkyl radical, a benzyl radical or a second Z group identical to or different from the first Z group;
• **R**_{**11**} represents one of the two valences of a connecting arm B; a C₁-C₆ alkyl radical; a monohydroxy(C₁-C₆)alkyl radical, a polyhydroxy(C₂-C₆)alkyl radical; an aryl radical; a benzyl radical; an amino(C₁-C₆)alkyl radical; an amino(C₁-C₆)alkyl radical, the amine of which is protected by a (C₁-C₆)alkylcarbonyl, carbamoyl or (C₁-C₆)alkylsulphonyl radical; a carboxy(C₁-C₆)alkyl radical; a cyano(C₁-C₆)alkyl radical; a carbamoyl(C₁-C₆)alkyl radical; a trifluoro (C₁-C₆)alkyl radical; a tri(C₁-C₆)alkylsilyl(C₁-C₆)alkyl radical; a sulphonamido(C₁-C₆)alkyl radical; a (C₁-C₆)alkylcarboxy (C₁-C₆)alkyl radical; a (C₁-C₆)alkylsulphinyl(C₁-C₆)alkyl radical; a (C₁-C₆)alkylsulphonyl(C₁-C₆)alkyl radical; a (C₁-C₆)alkylketo(C₁-C₆)alkyl radical; an N-[(C₁-C₆)alkyl]carbamoyl(C₁-C₆)alkyl radical; an N-[(C₁-C₆)alkyl]sulphonamido(C₁-C₆)alkyl radical;
• **x** and **y** are integers equal to 0 or 1; with the following conditions:
- in the unsaturated cationic groups of formula (II):
- when x = 0, the connecting arm D is attached to the nitrogen atom,
- when x = 1, the connecting arm D is attached to one of the ring members E, G, J or L,
- y can take the value 1 only:
1) when the ring members E, G, J and L simultaneously represent a carbon atom and when the R₇ radical is carried by the nitrogen atom of the unsaturated ring; or else
2) when at least one of the ring members E, G, J and L represents a nitrogen atom to which the R₇ radical is attached;
- in the unsaturated cationic groups of formula (III) :
- when x = 0, the connecting arm D is attached to the nitrogen atom,
- when x = 1, the connecting arm D is attached to one of the ring members E, G, J, L or M,
- y can take the value 1 only when at least one of the ring members E, G, J, L or M represents a divalent atom and when the R₇ radical is carried by the nitrogen atom of the unsaturated ring;
• **X**^{**-**} represents a monovalent or divalent anion,
**it being understood that:**
- (i) the number of cationic groups Z is at least equal to 1.

2. Cationic aminodianthraquinones according to Claim 1, **characterized in that** the rings of the unsaturated groups Z of formula (II) are chosen from pyrrole, imidazole, pyrazole, oxazole, thiazole and triazole rings.

3. Cationic aminodianthraquinones according to Claim 1, **characterized in that** the rings of the unsaturated groups Z of formula (III) are chosen from pyridine, pyrimidine, pyrazine, oxazine and triazine rings.

4. Cationic aminodianthraquinones according to any one of the preceding claims, **characterized in that** X⁻ is chosen from a halogen atom, a hydroxide, a hydrogen sulphate or a (C₁-C₆)alkyl sulphate.

5. Cationic aminodianthraquinones according to any one of the preceding claims, **characterized in that** they are chosen from those of following formulae (I)₂ to (I₅) and (I₉) to (I)₁₁:

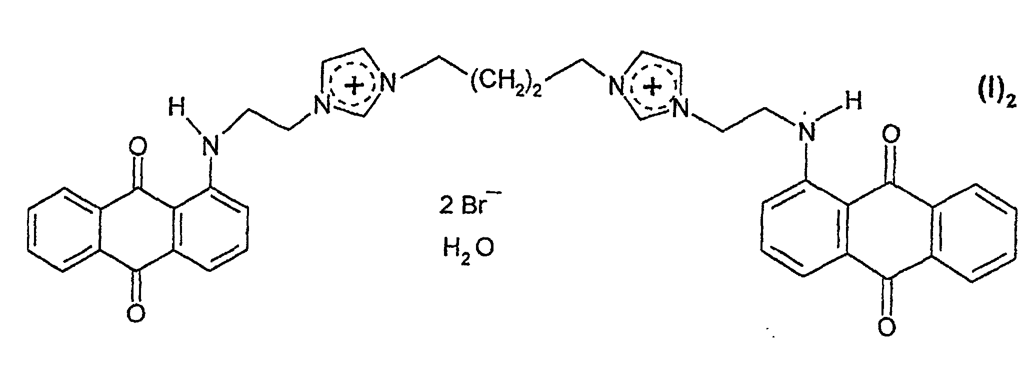

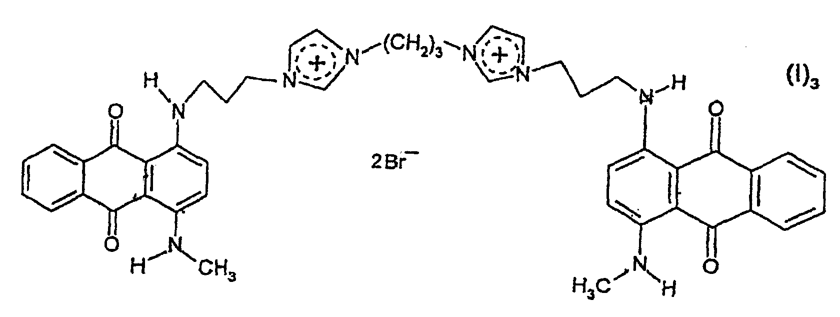

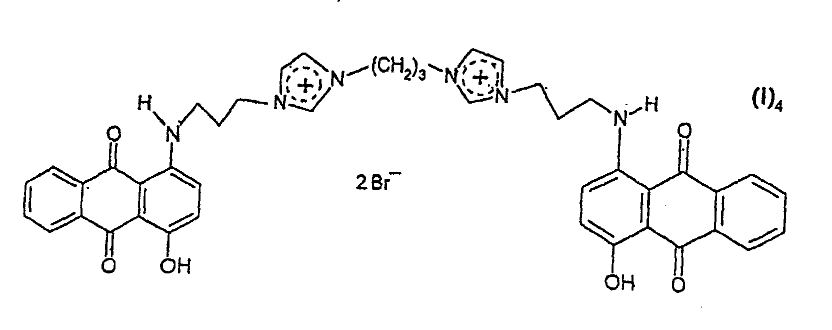

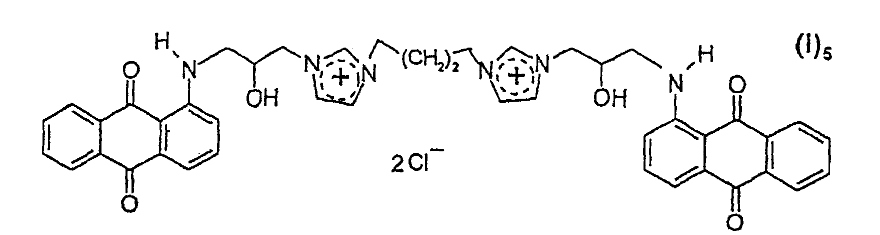

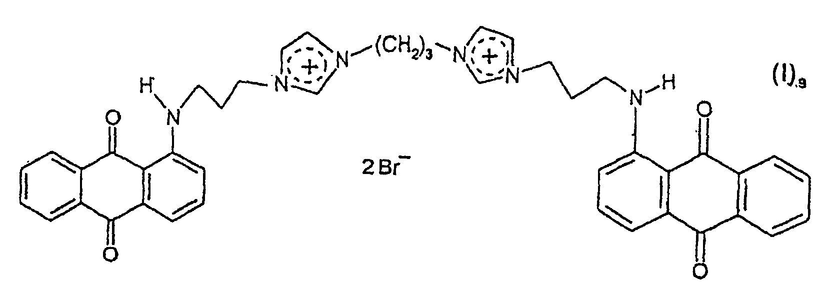

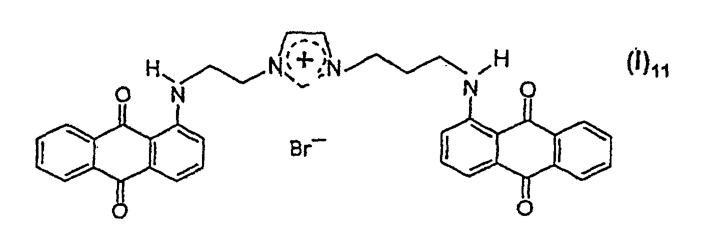

6. Cationic aminodianthraquinones according to any one of the preceding claims, **characterized in that** the addition salts with an acid are chosen from hydrochlorides, hydrobromides, sulphates, citrates, succinates, tartrates, lactates and acetates.

7. Use of cationic aminodianthraquinones of following formula (I): 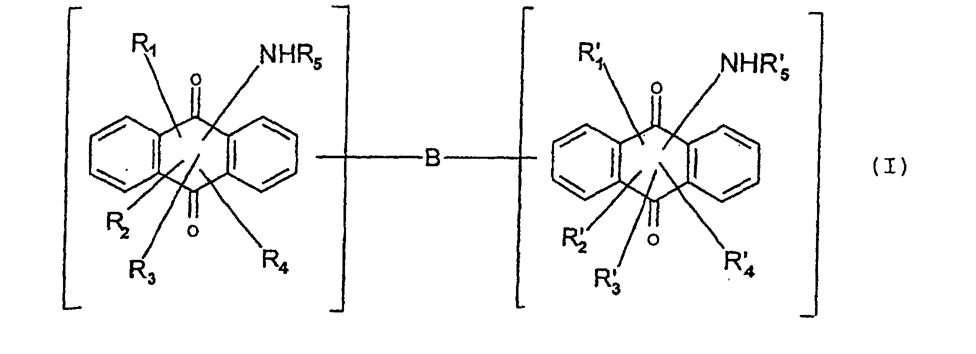 for which B, R₁, R₂, R₃, R₄, R₅, R'₁, R'₂, R'₃, R'₄ and R'₅ have the same meanings as in Claim 1,
as direct dyes in or for the manufacture of dyeing compositions for human keratinous substances, in particular for human keratinous fibres and more particularly the hair.

8. Dyeing composition for human keratinous substances, in particular for human keratinous fibres and more particularly the hair, **characterized in that** it comprises, in a medium appropriate for dyeing, an effective amount of at least one cationic aminodidianthraquinone of formula (I) defined in any one of Claims 1 to 7.

9. Composition according to Claim 8, **characterized in that** it has a pH of between 3 and 12.

10. Composition according to either one of Claims 8 and 9, **characterized in that** the cationic aminodianthraquinones are present in a concentration ranging from 0.005 to 12% by weight with respect to the total weight of the composition.

11. Composition according to Claim 10, **characterized in that** the cationic aminodianthraquinones are present in a concentration ranging from 0.05 to 6% by weight with respect to the total weight of the composition.

12. Composition according to any one of Claims 8 to 11, **characterized in that** it comprises other direct dyes chosen from nitrobenzene dyes, such as nitrophenylenediamines, nitrodiphenylamines, nitroanilines, nitrophenol ethers or nitrophenols, or nitropyridines, anthraquinone dyes other than those of formula (I), mono- or diazo, triarylmethane, azine, acridine or xanthene dyes, or metalliferous dyes.

13. Composition according to any one of Claims 8 to 12, **characterized in that** the medium appropriate for dyeing is an aqueous medium composed of water and/or organic solvents chosen from alcohols, glycols and glycol ethers in proportions of between 1 and 40% by weight with respect to the total weight of the composition.

14. Process for dyeing human keratinous fibres and in particular the hair by direct dyeing, **characterized in that** the dyeing composition defined in any one of Claims 8 to 13 is applied to dry or wet fibres and **in that** these fibres are dried without intermediate rinsing.

15. Process for dyeing human keratinous fibres and in particular the hair by direct dyeing, **characterized in that** the dyeing composition defined in any one of Claims 8 to 13 is applied to dry or wet fibres and **in that**, after having allowed the composition to act for 3 to 60 minutes approximately, the fibres are rinsed, are optionally washed, are rinsed again and are then dried.

## Patentansprüche

1. Kationische Aminodianthrachinone der folgenden Formel (I) und ihre Additionssalze mit einer Säure: 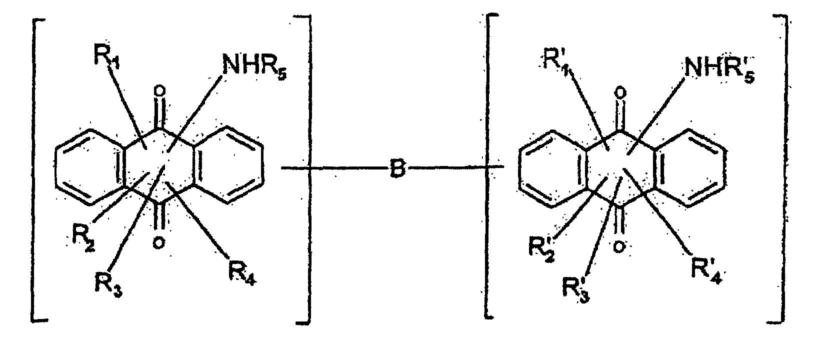 wobei in der Formel:
• B eine Verbindungsgruppe ist, die eine geradkettige oder verzweigte Alkylgruppe mit vorzugsweise 1 bis 14 Kohlenstoffatomen bedeutet, die durch eine oder mehrere (nachstehend definierte) Gruppen Z und/oder ein oder mehrere Heteroatome, wie Sauerstoffatome, Schwefelatome oder Stickstoffatome unterbrochen sein kann, gegebenenfalls mit einer oder mehreren Hydroxygruppen oder C₁₋₆-Alkoxy substituiert sein kann und eine oder mehrere Ketofunktionen enthalten kann;
• R₁, R₂, R₃, R₄, R'₁, R'₂, R'₃ und R'₄, die identisch oder voneinander verschieden sein können, eine der beiden Valenzen der Verbindungsgruppe B, Wasserstoff; Halogen; eine nachfolgend definierte Gruppe Z; C₁₋₆-Alkyl; C₁₋₆-Monohydroxyalkyl; C₂₋₆-Polyhydroxyalkyl; Cyano; Nitro; Carboxy; Carbamoyl; Sulfo; eine substituierte oder unsubstituierte Aminogruppe, die die nachstehend angegebenen Bedeutungen der Gruppe NHR₅ oder NHR'₅ aufweist und gleich oder verschieden sein kann; eine nachfolgend definierte Gruppe OR₆ oder SR₆ oder OR'₆ oder SR'₆ bedeuten;
• R₅ und R'₅, die gleich oder verschieden sind, eine der beiden Valenzen der Verbindungsgruppe B, Wasserstoff; eine nachfolgend definierte Gruppe Z; C₁₋₆-Alkyl; C₁₋₆-Monohydroxyalkyl; C₂₋₆-Polyhydroxyalkyl; Alkoxy(C₁₋₆)alkyl(C₁₋₆); Benzyl; C₁₋₆-Cyanoalkyl; C₁₋₆-Carbamoylalkyl; N-Alkyl(C₁₋₆)carbamoylalkyl(C₁₋₆); N,N-Dialkyl(C₁₋₆)carbamoylalkyl(C₁₋₆); C₁₋₆-Thiocarbamoylalkyl; C₁₋₆-Trifluoralkyl; C₁₋₆-Sulfoalkyl; Alkyl(C₁₋₆)carboxyalkyl(C₁₋₆); Alkyl(C₁₋₆)sulfinylalkyl(C₁₋₆); C₁₋₆-Aminosulfonylalkyl; N-Z-C₁₋₆-Aminosulfonylalkyl; N-Alkyl(C₁₋₆)aminosulfonylalkyl(C₁₋₆); N,N-Dialkyl(C₁₋₆)-aminosulfonylalkyl(C₁₋₆); Alkyl(C₁₋₆)carbonylalkyl(C₁₋₆); C₁₋₆-Aminoalkyl, wobei die Alkylgruppe mit einer oder mehreren Hydroxygruppen substituiert sein kann; C₁₋₆-Aminoalkyl, wobei die Alkylgruppe mit einer oder mehreren Hydroxygruppen substituiert sein kann und wobei die Aminogruppe mit einer oder zwei Gruppen substituiert ist, die identisch oder voneinander verschieden sind und unter den folgenden Gruppen ausgewählt sind: C₁₋₆-Alkyl, C₁₋₆-Monohydroxyalkyl, C₂₋₆-Polyhydroxyalkyl, Alkyl-(C₁₋₆)carbonyl, Carbamoyl, N-Alkyl(C₁₋₆)carbamoyl, N,N-Dialkyl(C₁₋₆)carbamoyl, Alkyl(C₁₋₆)sulfonyl, Formyl, Trifluoralkyl(C₁₋₆)carbonyl, Alkyl(C₁₋₆)carboxy, Thiocarbamoyl oder den Gruppen Z (nachstehend definiert) bedeuten oder gemeinsam mit dem Stickstoffatom, an das sie gebunden sind, einen 5- oder 6-gliedrigen Ring bilden können, der nur Kohlenstoffatome aufweist oder auch ein oder mehrere Heteroatome enthält;
• R₆ und R'₆, die gleich oder verschieden sind, eine der beiden Valenzen der Verbindungsgruppe B, Wasserstoff; C₁₋₆-Alkyl; C₁₋₆-Monohydroxyalkyl; C₂₋₆-Polyhydroxyalkyl; eine Gruppe Z (nachstehend definiert); Alkoxy(C₁₋₆)alkyl-(C₁₋₆); Aryl; Benzyl; C₁₋₆-Carboxyalkyl; Alkyl(C₁₋₆)carboxyalkyl(C₁₋₆); C₁₋₆-Cyanoalkyl; C₁₋₆-Carbamoylalkyl; N-Alkyl(C₁₋₆)carbamoylalkyl(C₁₋₆); N,N-Dialkyl(C₁₋₆)-carbamoylalkyl(C₁₋₆); C₁₋₆-Trifluoralkyl; C₁₋₆-Aminosulfonylalkyl; N-Z-C₁₋₆-Amino-sulfonylalkyl; N-Alkyl(C₁₋₆)aminosulfonylalkyl(C₁₋₆); N,N-Dialkyl-(C₁₋₆)aminosulfonylalkyl(C₁₋₆); Alkyl(C₁₋₆)-sulfinylalkyl(C₁₋₆); Alkyl(C₁₋₆)sulfonylalkyl(C₁₋₆); Alkyl(C₁₋₆)carbonylalkyl(C₁₋₆); C₁₋₆-Aminoalkyl, wobei die Alkylgruppe mit einer oder mehreren Hydroxygruppen substituiert sein kann; C₁₋₆-Aminoalkyl, wobei die Alkylgruppe mit einer oder mehreren Hydroxygruppen substituiert sein kann und wobei die Aminogruppe mit einer oder zwei Gruppen substituiert ist, die identisch oder voneinander verschieden sind und unter den folgenden Gruppen ausgewählt sind: C₁₋₆-Alkyl, C₁₋₆-Monohydroxyalkyl, C₂₋₆-Polyhydroxyalkyl, Alkyl(C₁₋₆)carbonyl, Formyl, Trifluoralkyl(C₁₋₆)carbonyl, Alkyl(C₁₋₆)carboxy, Carbamoyl, N-Alkyl(C₁₋₆)carbamoyl, N,N-Dialkyl(C₁₋₆)carbamoyl, Thiocarbamoyl, Alkyl(C₁₋₆)sulfonyl oder den Gruppen Z (nachstehend definiert) bedeuten oder gemeinsam mit dem Stickstoffatom, an das sie gebunden sind, einen 5- oder 6-gliedrigen Ring bilden können, der nur Kohlenstoffatome enthält oder auch ein oder mehrere Heteroatome aufweist;
• Z unter den ungesättigten kationischen Gruppen der folgenden Formeln (II) und (III) und den gesättigten kationischen Gruppen der folgenden Formel (IV) ausgewählt ist: 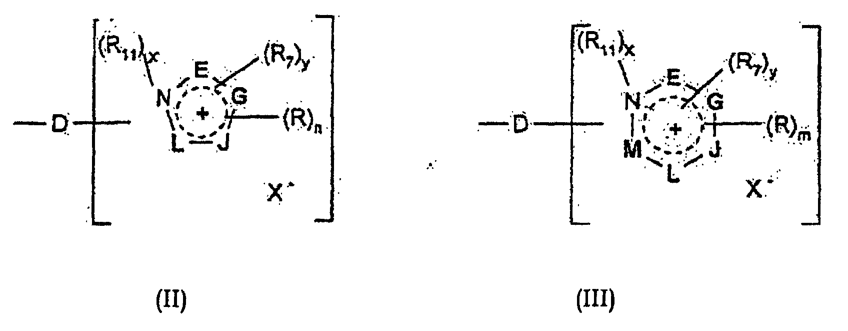 worin bedeuten:
• D eine Verbindungsgruppe, die eine Alkylkette mit vorzugsweise 1 bis 14 Kohlenstoffatome bedeutet, die geradkettig oder verzweigt vorliegt und mit einem oder mehreren Heteroatomen, wie beispielsweise Sauerstoff, Schwefel oder Stickstoff, unterbrochen sein kann, mit einer oder mehreren Hydroxygruppen oder C₁₋₆-Alkoxygruppen substituiert sein kann und eine oder mehrere Ketogruppen tragen kann;
• E, G, J, L und M, die identisch oder voneinander verschieden sind, ein Kohlenstoffatom, ein Sauerstoffatom, ein Schwefelatom oder ein Stickstoffatom;
• n 0 oder eine ganze Zahl von 1 bis 4;
• m 0 oder eine ganze Zahl von 1 bis 5;
• die Gruppen R, die identisch oder voneinander verschieden sind, eine der beiden Valenzen der Verbindungsgruppe B, eine zweite Gruppe Z, die identisch mit der ersten Gruppe Z oder von ihr verschieden sein kann, Halogen, Hydroxy, C₁₋₆-Alkyl, C₁₋₆-Monohydroxyalkyl, C₂₋₆-Polyhydroxyalkyl, Nitro, Cyano, C₁₋₆-Cyanoalkyl, C₁₋₆-Alkoxy, Trialkyl(C₁₋₆)silanalkyl(C₁₋₆), Amido, Aldehydo, Carboxy, C₁₋₆-Alkylcarbonyl, Thio, C₁₋₆-Thioalkyl, Alkyl(C₁₋₆)thio, Amino, eine Aminogruppe, die mit Alkyl(C₁₋₆)carbonyl, Carbamoyl oder Alkyl(C₁₋₆)sulfonyl geschützt ist; eine Gruppe NHR" oder NR"R"', worin R" und R"', die identisch oder voneinander verschieden sind, C₁₋₆-Alkyl, C₁₋₆-Monohydroxyalkyl oder C₂₋₆-Polyhydroxyalkyl bedeuten;
• R₇ eine der beiden Valenzen der Verbindungsgruppe B, C₁₋₆-Alkyl, C₁₋₆-Monohydroxyalkyl, C₂₋₆-Polyhydroxyalkyl, C₁₋₆-Cyanoalkyl, Trialkyl(C₁₋₆)silanalkyl(C₁₋₆), Alkoxy(C₁₋₆)alkyl(C₁₋₆), C₁₋₆-Carbamoylalkyl, Alkyl(C₁₋₆)carboxyalkyl(C₁₋₆), Benzyl oder eine zweite Gruppe Z, die identisch mit der ersten Gruppe Z oder von ihr verschieden ist;
• R₁₁ eine der beiden Valenzen der Verbindungsgruppe B, C₁₋₆-Alkyl; C₁₋₆-Monohydroxyalkyl; C₂₋₆-Polyhydroxyalkyl; Aryl; Benzyl; C₁₋₆-Aminoalkyl; C₁₋₆-Aminoalkyl, wobei die Aminogruppe mit Alkyl(C₁₋₆)carbonyl, Carbamoyl oder Alkyl(C₁₋₆)-sulfonyl geschützt ist; C₁₋₆-Carboxyalkyl; C₁₋₆-Cyanoalkyl; C₁₋₆-Carbamoylalkyl; C₁₋₆-Trifluoralkyl; Trialkyl(C₁₋₆)silanalkyl(C₁₋₆); C₁₋₆-Sulfonamidoalkyl; Alkyl(C₁₋₆)carboxyalkyl(C₁₋₆); Alkyl(C₁₋₆)-sulfinylalkyl(C₁₋₆); Alkyl(C₁₋₆)sulfonylalkyl(C₁₋₆); Alkyl(C₁₋₆)-ketoalkyl(C₁₋₆); N- Alkyl(C₁₋₆)carbamoylalkyl(C₁₋₆); N-Alkyl(C₁₋₆)-sulfonamidoalkyl(C₁₋₆);
• x und y 0 oder 1; mit den folgenden Bedingungen:
- in den ungesättigten kationischen Gruppen der Formel (II):
- die Verbindungsgruppe D ist an das Stickstoffatom gebunden, wenn x = 0,
- die Verbindungsgruppe D ist an eines der Atome E, G, J oder L gebunden, wenn x = 1,
- y kann nur den Wert 1 annehmen, wenn:
1) die Atome E, G, J und L gleichzeitig ein Kohlenstoffatom bedeuten und die Gruppe R₇ von dem Stickstoffatom des ungesättigten Rings getragen wird; oder
2) mindestens eines der Atome E, G, J und L ein Stickstoffatom bedeutet, an das R₇ gebunden ist;
- in den ungesättigten kationischen Gruppen der Formel (III):
- die Verbindungsgruppe D an das Stickstoffatom gebunden ist, wenn x = 0,
- die Verbindungsgruppe D an eines der Atome E, G, J, L oder M gebunden ist, wenn x = 1,
- y nur den Wert 1 annehmen kann, wenn mindestens eines der Atome E, G, J, L und M ein zweiwertiges Atom bedeutet und die Gruppe R₇ von dem Stickstoffatom des ungesättigten Rings getragen wird;
• X⁻ ein einwertiges oder zweiwertiges Anion;
mit der Maßgabe, daß:
- die Anzahl der kationischen Gruppen Z mindestens 1 ist.

2. Kationische Aminodianthrachinone nach Anspruch 1, **dadurch gekennzeichnet, daß** die Ringe der ungesättigten Gruppen Z der Formel (II) unter den Pyrrolringen, Imidazolringen, Pyrazolringen, Oxazolringen, Thiazolringen und Triazolringen ausgewählt sind.

3. Kationische Aminodianthrachinone nach Anspruch 1, **dadurch gekennzeichnet, daß** die Ringe der ungesättigten Gruppen Z der Formel (III) unter den Pyridinringen, Pyrimidinringen, Pyrazinringen, Oxazinringen und Triazinringen ausgewählt sind.

4. Kationische Aminodianthrachinone nach einem der vorhergehenden Ansprüche, **dadurch gekennzeichnet, daß** X⁻ Halogen, Hydroxid, Hydrogensulfat oder Alkyl(C₁₋₆)sulfat bedeutet.

5. Kationische Aminodianthrachinone nach einem der vorhergehenden Ansprüche, **dadurch gekennzeichnet, daß** sie unter den Verbindungen der folgenden Formeln (I)₂ bis (I)₁₂ ausgewählt sind:

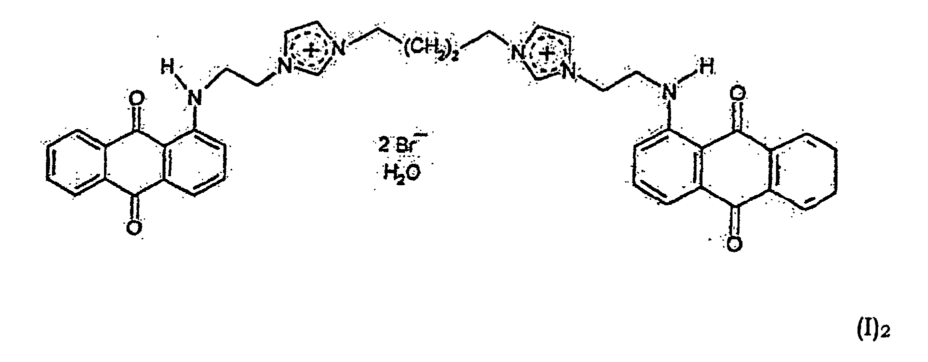

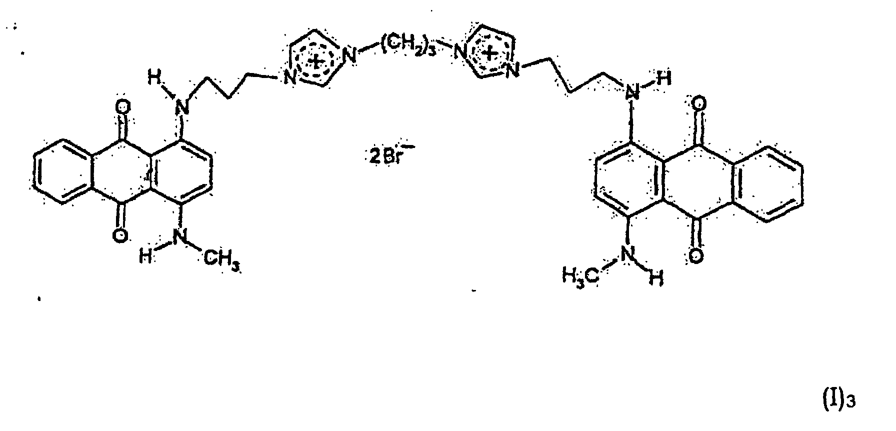

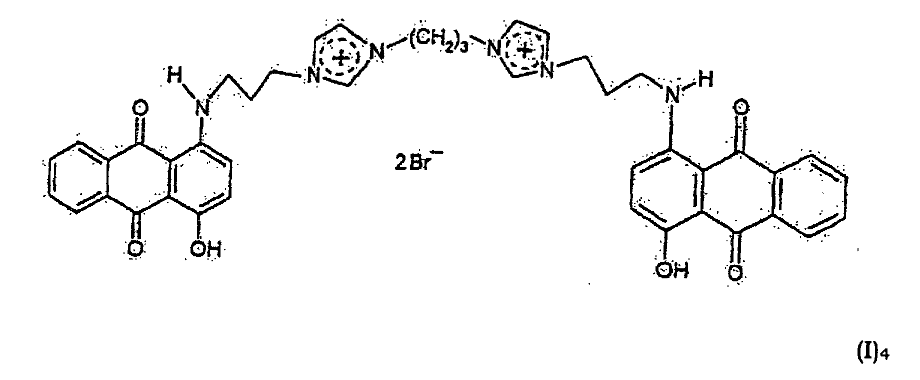

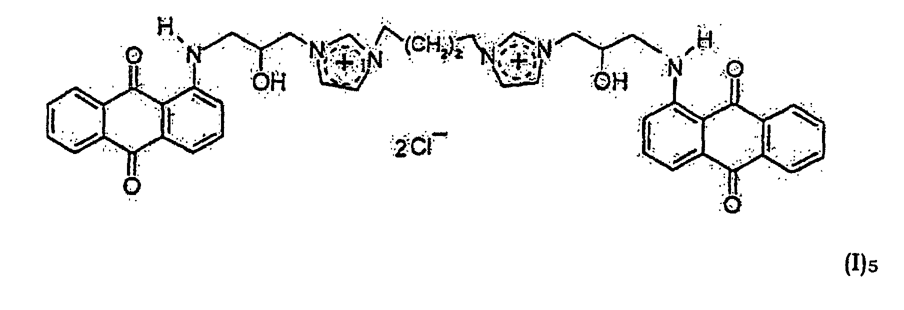

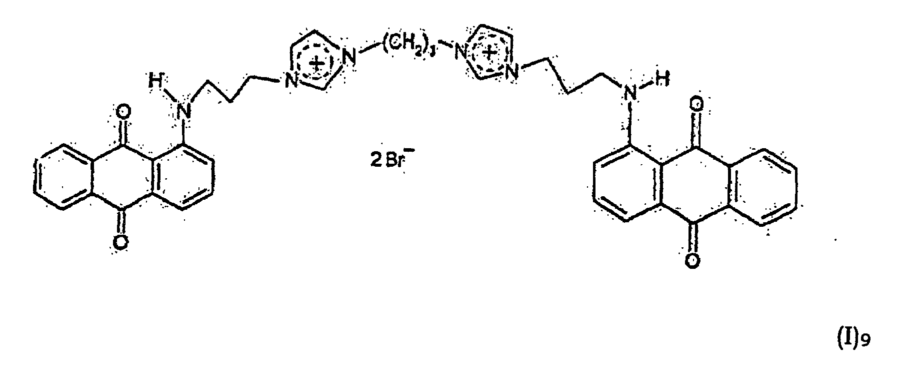

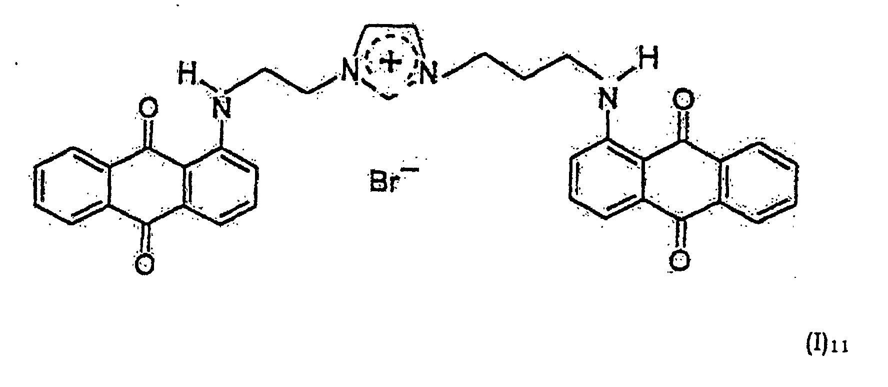

6. Kationische Aminodianthrachinone nach einem der vorhergehenden Ansprüche, **dadurch gekennzeichnet, daß** die Additionssalze mit einer Säure unter den Hydrochloriden, Hydrobromiden, Sulfaten, Citraten, Succinaten, Tartraten, Lactaten und Acetaten ausgewählt sind.

7. Verwendung von kationischen Aminodianthrachinonen der folgenden Formel (I): 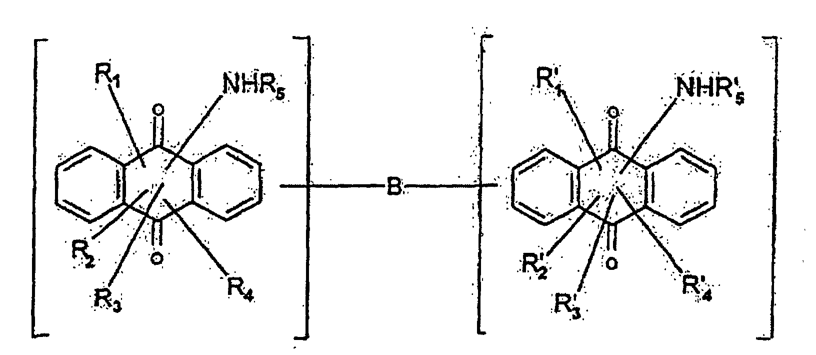 worin die Gruppen B, R₁, R₂, R₃, R₄, R'₁, R'₂, R'₃, R'₄ und R'₅ die in Anspruch 1 angegebenen Bedeutungen aufweisen, als Direktfarbstoffe in Farbmittelzusammensetzungen oder zur Herstellung von Farbmittelzusammensetzungen zum Färben von menschlichen Keratinsubstanzen und insbesondere menschlichen Keratinfasern, wie dem Haar.

8. Zusammensetzung zum Färben von menschlichen Keratinsubstanzen, insbesondere menschlichen Keratinfasern und besonders dem Haar, **dadurch gekennzeichnet, daß** sie in einem zum Färben geeigneten Medium mindestens ein kationisches Aminodianthrachinon der Formel (I) nach einem der Ansprüche 1 bis 7 in einer wirksamen Menge enthält.

9. Zusammensetzung nach Anspruch 8, **dadurch gekennzeichnet, daß** sie einen pH-Wert im Bereich von 3 bis 12 aufweist.

10. Zusammensetzung nach einem der Ansprüche 8 bis 9, **dadurch gekennzeichnet, daß** die kationischen Aminodianthrachinone in einer Konzentration von 0,005 bis 12 Gew.-%, bezogen auf das Gesamtgewicht der Zusammensetzung, vorliegen.

11. Zusammensetzung nach Anspruch 10, **dadurch gekennzeichnet, daß** die kationischen Aminodianthrachinone in einer Konzentration von 0,05 bis 6 Gew.-%, bezogen auf das Gesamtgewicht der Zusammensetzung, vorliegen.

12. Zusammensetzung nach einem der Ansprüche 8 bis 11, **dadurch gekennzeichnet, daß** sie weitere Direktfarbstoffe enthält, die unter den Nitrobenzol-Farbstoffen, wie Nitrophenylendiaminen, Nitrodiphenylaminen, Nitroanilinen, Nitrophenolethern oder Nitrophenolen, Nitropyridinen, Anthrachinon-Farbstoffen, die von den Verbindungen der Formel (I) verschieden sind, Mono- oder Diazofarbstoffen, Triarylmethan-Farbstoffen, Azin-Farbstoffen, Acridin-Farbstoffen und Xanthen-Farbstoffen oder metallhaltigen Farbstoffen ausgewählt sind.

13. Zusammensetzung nach einem der Ansprüche 8 bis 12, **dadurch gekennzeichnet, daß** das zum Färben geeignete Medium ein wäßriges Medium ist, das aus Wasser und/oder organischen Lösungsmitteln in Mengenanteilen von 1 bis 40 Gew.-%, bezogen auf das Gesamtgewicht der Zusammensetzung, besteht, die unter den Alkoholen, Glykolen und Glykolethern ausgewählt sind.

14. Verfahren zum Färben von menschlichen Keratinfasern und insbesondere dem Haar durch direktes Färben, **dadurch gekennzeichnet, daß** eine Farbmittelzusammensetzung nach einem der Ansprüche 8 bis 13 auf die trockenen oder feuchten Keratinfasern aufgetragen wird und die Fasern ohne zwischenzeitliches Spülen getrocknet werden.

15. Verfahren zum Färben von menschlichen Keratinfasern und insbesondere dem Haar durch direktes Färben, **dadurch gekennzeichnet, daß** eine Farbmittelzusammensetzung nach einem der Ansprüche 8 bis 13 auf die trockenen oder feuchten Keratinfasern aufgetragen wird und nach einer Einwirkzeit von etwa 3 bis 60 min die Fasern gespült, gegebenenfalls gewaschen, nochmals gespült und getrocknet werden.
